# NEW EUROPEAN PATENT SPECIFICATION

(11) **EP 1 513 845 B2**
(45) Date of publication and mention of the opposition decision: **03.11.2010**
(45) Mention of the grant of the patent: 19.09.2007
(21) Application number: 03732782.2
(22) Date of filing: 10.06.2003
(51) Int. Cl.: C07D 495/04

(54) **A PROCESS FOR THE PREPARATION OF OLANZAPINE AND AN INTERMEDIATE THEREFOR**
VERFAHREN ZUR HERSTELLUNG VON OLANZAPIN UND EIN ZWISCHENPRODUKT DAFÜR
PROCEDE DE PREPARATION D'OLANZAPINE ET INTERMEDIAIRE UTILISE

(30) Priority: 20.06.2002 PL 35464202
(43) Date of publication of application: 16.03.2005
(60) Divisional application: 06100356.2
(73) Proprietor: Adamed SP. Z O.O., 05-152 Czosnów k/Warszawy (PL)
(72) Inventor: MAJKA, Zbigniew, 39-102 Lubzina (PL); STAWINSKI, Tomasz, 05-462 Wiazowna (PL); RECHNIO, Justyna, 05-530 Gòra Kalwaria (PL); WIECZOREK, Maciej, 05-092 Lomianki (PL)
(74) Representative: HOFFMANN EITLE
(86) International application number: PCT/IB2003/002181
(87) International publication number: WO 2004/000847

(56) References cited:
- EP-A- 0 454 436
- EP-A2- 0 429 996
- WO-A1-93/22293
- GB-A- 1 533 235
- US-A- 3 950 425
- US-A- 4 115 568
- CALLIGARO D O ET AL: "The synthesis and biological activity of some known and putative metabolites of the atypical antipsychotic agent olanzapine (LY170053)" BIOORGANIC & MEDICINAL CHEMISTRY LETTERS, OXFORD, GB, vol. 7, no. 1, 7 January 1997 (1997-01-07), pages 25-30, XP004135960 ISSN: 0960-894X
- ' Synthesis of Olanzapine' CHINESE JOURNAL OF PHARMACEUTICALS 2001 vol. 32, no. 9, 2001 - 2001, pages 391 - 393
- JOURNAL OF MEDICAL CHEMISTRY vol. 23, no. 8, 1980 - 1980, pages 884 - 889

## Description

### Technical field

The invention relates to a process for olanzapine preparation, in particular to the process for olanzapine preparation using *N-*demethylolanzapine as a starting material.

### Background of the invention

Olanzapine, or 2-methyl-4-[4-methyl-1-piperazinyl]-10H-thieno[2,3-*b*][1,5]-benzodiazepine, is a recognised drug acting on the central nervous system and is known, among others, from EP 0454436.

In EP 0454436 there are disclosed processes for olanzapine preparation. One of the known procedures consists in reduction and cyclization reaction of 2-(2-nitroanilino)-5-methylthiophen-3-carbonitrile with stannous chloride SnCl₂ in an aqueous-alcoholic solution of hydrogen chloride, followed by a reaction of thus formed 4-amino-2-methyl-10H-thieno[2,3-b][1,5]-benzodiazepine with *N-*methylpiperazine in an organic solvent such as anisole, toluene, dimethylformamide or dimethylsulphoxide (DMSO), preferably at a temperature from 100 to 150°C, to produce olanzapine (see also Bioorg. Med. Chem. Lett., Vol. 7, pp. 25-30, 1997). Another of the known procedures consists in cyclization of 1-{[2-(2-aminoaniline)-5-methylthiophen-3-yl] carbonyl}-4-methylpiperazine, which is in turn obtained from methyl cyanoacetate in a series of laborious steps requiring specific complex reaction conditions, reactants and reducing agents as well as high-boiling and hard to remove solvents, like toluene, DMF, DMSO, etc.. The reaction yields of the prior-art processes are not high. Further disadvantage of the prior-art processes is generation of impurities which have to be removed by repeated crystallizations, what has adverse effect on the process efficiency.

The aim of this invention was to develop a new process for olanzapine preparation, which would not require the use of hard-to-remove organic solvents.

Further aim was to develop a new process for olanzapine preparation, which would involve more simple chemical procedures, while allowing to obtain high yields of the final product having satisfactory purity.

To remove the aforementioned deficiencies of the prior art the applicants have developed new processes for olanzapine preparation, wherein *N*-demethylolanzapine, that is, 2-methyl-4-piperazin-1-yl-10H-thieno[2,3-*b*][1,5]benzodiazepine is used as the starting material, which is subjected to N-methylation to form olanzapine.

According to the processes of the invention crude olanzapine is produced which is as pure as the one obtained by prior art processes, in mild conditions with relatively short reaction times and low reaction temperatures. The use of low-volatile solvents and generation of great amounts of impurities is avoided.

*N*-Demethylolanzapine is a compound known as an olanzapine metabolite and is described by Calligaro et al., Biorg. & Med. Chem. Letters, 1, 25-39, (1997), and in the publication of the international patent application WO00/30650. This compound can readily be made by a reaction of the known compound 4-amino-2-methyl-10H-thieno[2,3-*b*][1,5]benzodiazepine with piperazine as described by Calligaro et al., Biorg. & Med. Chem. Letters, 1, 25-39, (1997).

### Detailed description of the invention

According to the invention, there is provided a process for the preparation of olanzapine of formula I 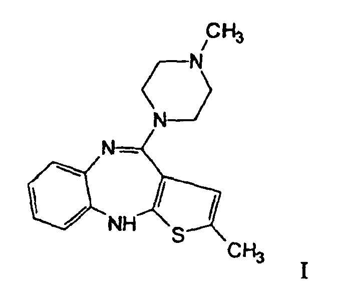 which comprises reductive *N-*methylation of 2-methyl-4-piperazin-1-yl-10H-thieno[2,3-*b*][1,5]benzodiazepine (*N*-demethylolanzapine) of formula II 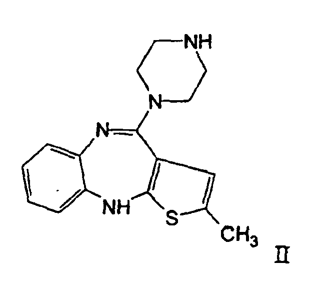 with formaldehyde in the presence of a reducing agent, wherein a borohydride of a Group I or II metal is used as said reducing agent and the reaction is performed in an aqueous medium at a temperature in the range from -10°C to +20°C.

An alkali-metal borohydride may be used, in particular sodium borohydride. The preferred reaction temperature range is zero to 5°C.

The reductive *N*-methylation using formaldehyde in the presence of a borohydride is carried out in a simple manner which is as such known in the art. Typically, the reaction is performed in an aqueous solution in the presence of acetic acid and sodium acetate as a buffer. Into the reaction mixture containing *N*-demethylolanzapine and formaldehyde upon its cooling the borohydride is added portionwise. After completion of the reaction the mixture is made alkaline, thereby the crude olanzapine precipitates and the precipitate is subsequently isolated.

Alternatively, in the second variant of the process (not in accordance with the invention), said reductive *N*-methylation with formaldehyde can also be carried out using formic acid as said reducing agent.

The *N*-methylation with formaldehyde in formic acid is generally known as the Eschweiler-Clarke reaction. The procedure is simple and is known in the art as such. In general, for one mole of olanzapine about 1.25 mole of formaldehyde is taken (in a little excess because of formaldehyde volatility) and 2-4 moles of formic acid which serves here also as a solvent in the reaction. Formaldehyde can also be used in the form of a 35-% formalin. The reaction is carried out in an aqueous solution at the reflux temperature of the reaction mixture. After completion of the reaction, the reaction mixture is allowed to cool thereby crude olanzapine precipitates.

In the third variant of the process (not in accordance with the invention), the said reductive *N-*methylation with formaldehyde can alternatively be carried out by low- or medium-pressure catalytic hydrogenation with hydrogen in the presence of a metal catalyst. As a metal catalyst can be used, for instance, platinum, palladium and nickel catalysts, e.g. Pd/C, Pt/C or Raney's nickel. The catalytic hydrogenation reaction in the presence of metallic catalysts is as such well known in the art and is described in handbooks.

The invention will be described in more detail with reference to the following, non-limiting examples, which should not be construed as the limitation of its scope.

### Example 1.

### N-Demethylolanzapine Preparation

A mixture of 4-amino-2-methyl-10H-thieno[2,3-*b*][1,5]benzodiazepine hydrochloride (3 g, 11.3 mmol), piperazine (7 g, 81.4 mmol) in 15 ml of DMSO and 15 ml of toluene was heated to reflux under an inert gas atmosphere, with no access of moisture of the air. The reaction mixture was refluxed for 2 h.

After completion of the reaction the reaction mixture was cooled in an ice-bath and 30 ml of distilled water was added. The mixture was stirred at 5°C for 1 h until completion of the formation of a precipitate. The light-yellow precipitate was filtered off, washed with water and dried in a vacuum desiccator over silica gel. 2.89 g (yield 85,7%) of *N*-Demethylolanzapine was obtained; m.p. 144.5°C.

### Example 2

### Olanzapine Preparation by Reductive Alkylation of N-Demethylolanzapine with Formaldehyde in the Presence of Sodium Borohydride

A mixture of N-demethylolanzapine (10 g, 33.5 mmol), anhydrous sodium acetate (6.6 g, 80 mmol) (or an equivalent amount of the acetate hydrate), 34 ml glacial acetic acid, 20 ml of 37-% aqueous formalin and 100 ml of distilled water was cooled to 0°C. Subsequently, sodium borohydride (8.5 g, 0.22 mmol) was added in small portions while maintaining temperature at 0°C and with vigorous stirring. Upon addition of the total amount of the borohydride the foaming solution was stirred at a low temperature for 1 h, then it was made alkaline to a pH = ca. 9 with 2N NaOH aq. The light-yellow precipitate produced was filtered off and washed with water. The crude product was dried in a circulating-air oven at ca. 25°C. 10.2 g of olanzapine was obtained with a purity 97% as determined by HPLC (yield: 97,3%).

### Example 3

### Olanzapine Preparation by Reductive N-Methylation with Formaldehyde in Formic Acid (the Eschweiler-Clarke Reaction) (not in accordance with the invention)

A mixture of 20 ml of 85-% formic acid and 14 ml of an 37-% aqueous formaldehyde was cooled in an ice-bath to 0°C. While cooling *N*-demethylolanzapine (50 g, 0.168 mol) was added under vigorous stirring. After addition of the amine the mixture was heater to reflux in an oil bath and maintained under reflux for 8 h. Then the mixture was cooled in an ice-bath and gently made alkaline to a pH = ca. 8 with 2N NaOH aq. The yellow precipitate formed was filtered off, washed with water and dried. Crude olanzapine was obtained (40 g) of a purity of 95% by HPLC (yield: 76,2%).

## Claims

1. A process for the preparation of olanzapine of formula I 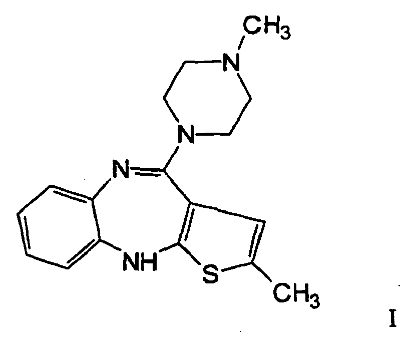 which process comprises reductive N*-*methylation of 2-methyl-4-piperazin-1-yl-10H-thieno[2,3-*b*][1,5]benzodiazepine (*N*-demethyl-olanzapine) of formula II 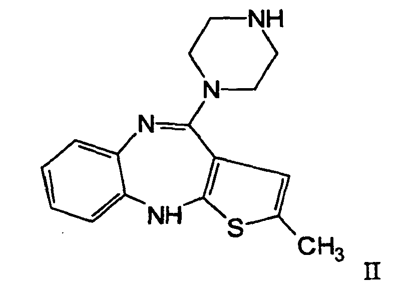 with formaldehyde in the presence of a reducing agent, wherein said reductive N-methylation is performed with a Group I or II metal borohydride as a reducing agent in an aqueous environment at a temperature in the range from -10°C to +20°C.

2. The process according to Claim 1, wherein the borohydride is an alkaline-metal borohydride, in particular sodium borohydride.

3. The process according to Claim 1 or 2, wherein the said reductive N-methylation is performed at a temperature in the range from 0°C to 5°C.

## Patentansprüche

1. Verfahren zur Herstellung von Olanzapin der Formel (I) 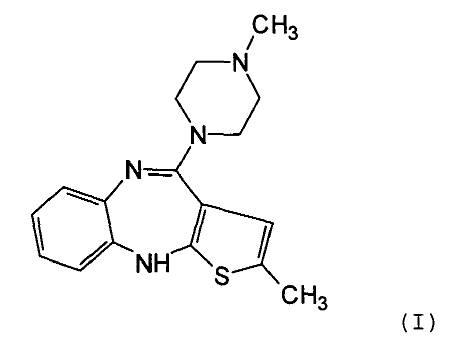 wobei das Verfahren die reduktive N-Methylierung von 2-Methyl-4-piperazin-1-yl-10H-thieno[2,3-b][1,5]-benzodiazepin (N-Desmethylolanzapin) der Formel (II) 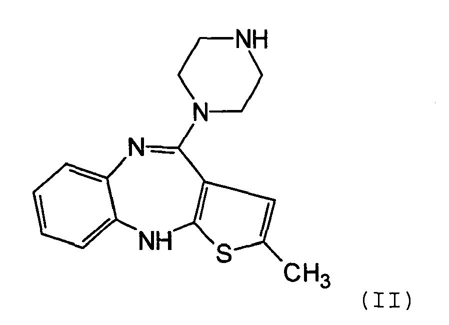 mit Formaldehyd in Gegenwart eines Reduktionsmittels umfasst, wobei die reduktive N-Methylierung mit einem Metallborhydrid der Gruppe I oder II als Reduktionsmittel in einem wässrigen Milieu bei einer Temperatur im Bereich von -10°C bis +20°C durchgeführt wird.

2. Verfahren gemäss Anspruch 1, wobei das Borhydrid ein Alkalimetallborhydrid, insbesondere Natriumborhydrid, ist.

3. Verfahren gemäss Anspruch 1 oder 2, wobei die reduktive N-Methylierung bei einer Temperatur im Bereich von 0°C bis 5°C durchgeführt wird.

## Revendications

1. Procédé pour la préparation de l'olanzapine de formule I 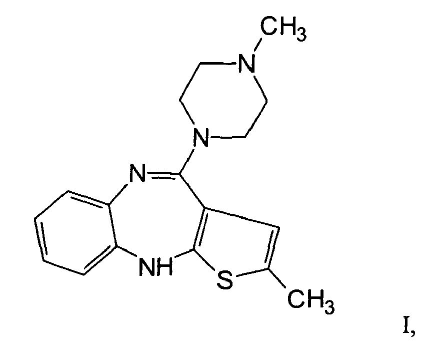 ledit procédé comprenant la N-méthylation réductrice de 2-méthyl-4-pipérazin-1-yl--10H-thiéno[2,3-b][1,5]benzodiazépine (N-desméthylolanzapine) de formule II 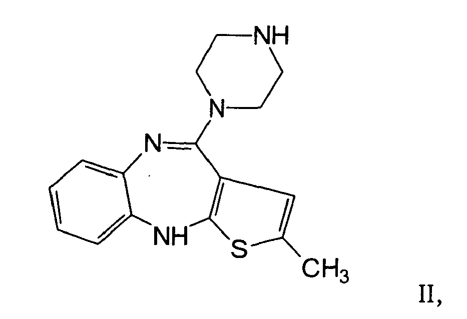 avec du formaldéhyde en présence d'un agent réducteur,
dans lequel ladite N-méthylation réductrice est effectuée avec un borohydrure de métal du Groupe I ou II en tant qu'agent réducteur dans un environnement aqueux à une température dans la plage de -10°C à +20°C.

2. Procédé selon la revendication 1, dans lequel le borohydrure est un borohydrure de métal alcalin, en particulier le borohydrure de sodium.

3. Procédé selon les revendications 1 ou 2, dans lequel ladite N-méthylation réductrice est effectuée à une température dans la plage de 0°C à 5°C.
